# EUROPÄISCHE PATENTSCHRIFT

(11) **EP 2 275 078 B1**
(45) Veröffentlichungstag und Bekanntmachung des Hinweises auf die Patenterteilung: **07.12.2016**
(21) Anmeldenummer: 10182140.3
(22) Anmeldetag: 10.08.2005
(51) Int. Cl.: A61K 8/44, A61K 8/42, A61K 8/64, A61Q 19/00, A61Q 19/08, A61K 8/67, A61K 8/36, A61K 8/55, A61K 8/73, A61K 8/63, A61K 8/99, A61Q 19/02

(54) **Kosmetische und dermatologische Zusammensetzungen mit (2-Hydroxyethyl)harnstoff**
Cosmetic and dermatological compositions comprising (2-hydroxyethyl)urea
Compositions cosmétiques et dermatologiques à base de (2-hydroxyéthyl)urée

(30) Priorität: 15.10.2004 DE 102004050563; 17.11.2004 DE 102004055541; 05.01.2005 DE 102005000868; 07.06.2005 DE 102005026357; 13.08.2004 DE 102004039550
(43) Veröffentlichungstag der Anmeldung: 19.01.2011
(62) Teilanmeldung aus: 05770877.8
(73) Patentinhaber: Henkel AG & Co. KGaA, 40589 Düsseldorf (DE)
(72) Erfinder: Döring, Thomas, Scottsdale, AZ 35255 (US); Träger, Anemone, 50968, Köln (DE); Waldmann-Laue, Marianne, 40789, Monheim (DE); Anderheggen, Bernd, 41189, Mönchengladbach (DE)

(56) Entgegenhaltungen:
- EP-A- 1 535 607
- WO-A-03/005979
- WO-A2-03/005980
- DE-A1- 2 703 185
- US-A- 6 008 246
- "Cosmetic skin-cream - contg. aloe, marigold, willow extracts, lactic acid, betaine-hydrochloride, urea, etc", DERWENT, 3. Februar 1990 (1990-02-03), XP002205036,

## Beschreibung

Die Erfindung betrifft topische kosmetische oder dermatologische Zusammensetzungen, die in einem geeigneten kosmetischen oder dermatologischen Träger eine Kombination von alkyl- oder hydroxyalkylsubstituierten Harnstoffen, insbesondere (2-Hydroxyethyl) - harnstoff, mit mindestens einem kosmetischen oder dermatologischen Wirkstoff, ausgewählt aus natürlichen Betainverbindungen, enthalten.

Topische Zusammensetzungen, die kosmetische oder dermatologische Wirkstoffe enthalten, sind im Stand der Technik bekannt. Ein häufig auftretender Nachteil ist allerdings die begrenzte Wirksamkeit, da die Wirkstoffe meist an der obersten Schicht der Epidermis verbleiben, deren Zellen bereits die letzte Stufe vor der Desquamation im Kreislauf der ständigen Erneuerung der Epidermis darstellen. Wünschenswert dagegen wäre zum Beispiel die Einschleusung der applizierten Wirkstoffe auch in tiefere Schichten der Epidermis, damit die Wirkstoffe der Haut insgesamt länger zur Verfügung stehen, um ihre maximale Wirkung erzielen zu können. Da die kosmetischen oder dermatologischen Wirkstoffe für einen beträchtlichen Anteil der Rohstoffkosten einer topischen Zusammensetzung verantwortlich sind, ist eine verbesserte Ausnutzung der Wirkstoffe, bevorzugt unter Ausnutzung von Synergieeffekten, von großem ökonomischen Interesse für den Hersteller.

Aufgabe der vorliegenden Erfindung war es, topische kosmetische oder dermatologische Zusammensetzungen mit einer optimierten Wirksamkeit der enthaltenen Wirkstoffe bereitzustellen.

Eine weitere Aufgabe der vorliegenden Erfindung war es, topische kosmetische oder dermatologische Zusammensetzungen zur nicht-therapeutischen, kosmetischen Behandlung und/oder Minimierung von Hautfalten und -fältchen und trockener Haut bereitzustellen. Alkyl- oder hydroxyalkylsubstituierte Harnstoffe, insbesondere (2-Hydroxyethyl)harnstoff, sind, ähnlich wie Harnstoff selbst, im Stand der Technik als feuchtigkeitsspendende und hautweichmachende Wirkstoff bekannt, beispielsweise aus DE 2703185 A1.

Überraschend und für den Fachmann nicht vorhersehbar wurde nun gefunden, dass alkyl- oder hydroxyalkylsubstituierte Harnstoffe, insbesondere (2-Hydroxyethyl)-harnstoff, auch in der Lage sein können, die Wirksamkeit ausgewählter kosmetischer oder dermatologischer Wirkstoffe zu optimieren.

Die US 6 008 246 A offenbart die Verwendung von Betain (Trimethylglycin) als Feuchtigkeitsspender bzw. Verbesserer des Hautzustandes.

In der EP 1 535 607 A werden Zusammensetzungen offenbart, die hydroxyalkylsubstituierte Harnstoffverbindungen und synergistische Moisturizer enthalten.

Gegenstand der vorliegenden Erfindung sind kosmetische oder dermatologische topische Zusammensetzungen, die in einem geeigneten kosmetischen oder dermatologischen Träger mindestens einen hydroxyalkylsubstituierten Harnstoff der allgemeinen Formel (A), 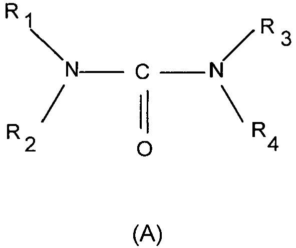 in der R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ - R₄ eine C₂-C₆-Hydroxyalkyl-Gruppe, die mit 1 bis 5 Hydroxylgruppen oder C₁-C₄-Hydroxyalkyl-Gruppen substituiert ist, darstellt,
sowie mindestens einen Wirkstoff, ausgewählt aus natürlichen Betainverbindungen, wobei die natürliche Betainverbindung ausgewählt ist aus Betain (Me₃N⁺-CH₂-COO⁻) mit Me = Methyl.

Erfindungsgemäß besonders bevorzugte Zusammensetzungen sind dadurch gekennzeichnet, dass der alkyl- oder hydroxyalkylsubstituierte Harnstoff gemäß Formel (A) ausgewählt ist aus (2-Hydroxyethyl)harnstoff.

Erfindungsgemäß besonders bevorzugte Zusammensetzungen sind dadurch gekennzeichnet, dass sie mindestens einen hydroxyalkylsubstituierten Harnstoff gemäß Formel (A), insbesondere (2-Hydroxyethyl)harnstoff, in einer Gesamtmenge von 0,01 - 50 Gew.-%, bevorzugt 0,1 - 20 Gew.-%, besonders bevorzugt 1 - 10 Gew.-% und außerordentlich bevorzugt 2 - 5 Gew.-%, bezogen auf die gesamte Zusammensetzung, enthalten. (2-Hydroxyethyl)harnstoff, bei Raumtemperatur ein kristalliner Feststoff, ist beispielsweise als Handelsprodukt Hydrovance der National Starch and Chemical Company als ca. 45 - 55 %ige wässrige Lösung erhältlich.

Die erfindungsgemäßen Zusammensetzungen enthalten neben dem mindestens einen hydroxyalkylsubstituierten Harnstoff gemäß Formel (A), insbesondere (2-Hydroxyethyl)harnstoff, mindestens eine natürliche Betainverbindung, die ausgewählt ist aus Betain (Me₃N⁺-CH₂-COO⁻) mit Me = Methyl.

Erfindungsgemäß besonders bevorzugte Zusammensetzungen sind dadurch gekennzeichnet, dass sie neben dem mindestens einen hydroxyalkylsubstituierten Harnstoff gemäß Formel (A), insbesondere (2-Hydroxyethyl)harnstoff, die mindestens eine natürliche Betainverbindung in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.

Ein weiterer Gegenstand der vorliegenden Erfindung ist die Verwendung einer kosmetischen oder dermatologischen topischen Zusammensetzung, die in einem geeigneten kosmetischen oder dermatologischen Träger mindestens einen hydroxyalkylsubstituierten Harnstoff der allgemeinen Formel (A), 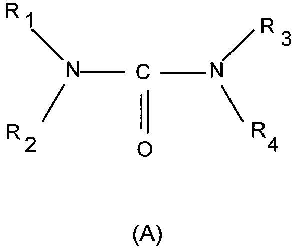 in der R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ - R₄ eine C₂-C₆-Hydroxyalkyl-Gruppe, die mit 1 bis 5 Hydroxylgruppen oder C₁-C₄-Hydroxyalkyl-Gruppen substituiert ist, darstellt, insbesondere (2-Hydroxyethyl)harnstoff, sowie mindestens einen Wirkstoff enthält, der ausgewählt ist aus natürlichen Betainverbindungen, wobei die natürliche Betainverbindung ausgewählt ist aus Betain (Me₃N⁺-CH₂-COO⁻) mit Me = Methyl. zur nicht-therapeutischen, kosmetischen Behandlung und/oder Minimierung von Hautfalten und -fältchen, den Anzeichen der intrinsischen und extrinsischen Hautalterung, trockener Haut.

Ein weiterer Gegenstand der vorliegenden Erfindung ist ein nicht-therapeutisches Verfahren zur nicht-therapeutischen, kosmetischen Behandlung und/oder Minimierung von Hautfalten und -fältchen, trockener Haut, das dadurch gekennzeichnet ist, dass eine kosmetische oder dermatologische topischen Zusammensetzung, die in einem geeigneten kosmetischen oder dermatologischen Träger mindestens einen hydroxyalkylsubstituierten Harnstoff der allgemeinen Formel (A), 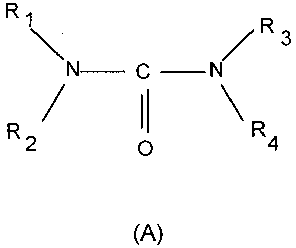 in der R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ - R₄ eine C₂-C₆-Hydroxyalkyl-Gruppe, die mit 1 bis 5 Hydroxylgruppen oder C₁-C₄-Hydroxyalkyl-Gruppen substituiert ist, darstellt, insbesondere (2-Hydroxyethyl)harnstoff, sowie mindestens einen Wirkstoff enthält, der ausgewählt ist aus natürlichen Betainverbindungen, wobei die natürliche Betainverbindung ausgewählt ist aus Betain (Me₃N⁺-CH₂-COO⁻) mit Me = Methyl, auf die Haut aufgetragen wird.

Vorteilhafterweise liegen die erfindungsgemäßen Hautbehandlungsmittel in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaumes, eines Puders oder einer Mischung mit mindestens einem als medizinischen Klebstoff geeigneten Polymer vor. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.

In einer besonderen Ausführungsform der erfindungsgemäßen Mittel liegen die Mittel als Mikroemulsion vor. Unter Mikroemulsionen werden im Rahmen der Erfindung neben den thermodynamisch stabilen Mikroemulsionen auch die sogenannten "PIT"-Emulsionen verstanden. Bei diesen Emulsionen handelt es sich um Systeme mit den 3 Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversiontemperatur oder "PIT" bezeichnet) Mikroemulsionen aus, die sich bei weiterer Erwärmung in Wasser-in-Öl-Emulsionen umwandeln. Bei anschließendem Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen.

In der Ausführungsform als Emulsion oder als tensidische Lösung, z. B. als Reinigungsmittel, enthalten die erfindungsgemäßen Zusammensetzungen mindestens eine oberflächenaktive Substanz als Emulgator oder Dispergiermittel. Geeignete Emulgatoren sind beispielsweise Anlagerungsprodukte von 4 bis 30 Mol Ethylenoxid und/oder 0 bis 5 Mol Propylenoxid an lineare C₈-C₂₂-Fettalkohole, an C₁₂-C₂₂-Fettsäuren und an C₈-C₁₅-Alkylphenole, C₁₂-C₂₂-Fettsäuremono- und -diester von Anlagerungsprodukten von 1 bis 30 Mol Ethylenoxid an C₃-C₆-Polyole, insbesondere an Glycerin, Ethylenoxid- und Polyglycerin-Anlagerungsprodukte an Methylglucosid-Fettsäureester, Fettsäurealkanolamide und Fettsäureglucamide, C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind, Gemische aus Alkyl-(oligo)-glucosiden und Fettalkoholen, z. B. das im Handel erhältliche Produkt Montanov^{®}68, Anlagerungsprodukte von 5 bis 60 Mol Ethylenoxid an Rizinusöl und gehärtetes Rizinusöl, Partialester von Polyolen mit 3-6 Kohlenstoffatomen mit gesättigten C₈-C₂₂-Fettsäuren, Sterole (Sterine), insbesondere Cholesterol, Lanosterol, Beta-Sitosterol, Stigmasterol, Campesterol und Ergosterol sowie Mykosterole, Phospholipide, vor allem Glucose-Phospolipide, Fettsäureester von Zuckern und Zuckeralkoholen wie Sorbit, Polyglycerine und Polyglycerinderivate, bevorzugt Polyglyceryl-2-dipolyhydroxystearat (Handelsprodukt Dehymuls^{®} PGPH) und Polyglyceryl-3-diisostearat (Handelsprodukt Lameform^{®} TGI) sowie lineare und verzweigte C₈-C₃₀-Fettsäuren und deren Na-, K-, Ammonium-, Ca-, Mg- und Zn - Salze.

Die erfindungsgemäßen Mittel enthalten die Emulgatoren bevorzugt in Mengen von 0,1 bis 25 Gew.-%, insbesondere 0,5 - 15 Gew.-%, bezogen auf das gesamte Mittel.

In einer besonders bevorzugten Ausführungsform ist mindestens ein nichtionischer Emulgator mit einem HLB-Wert von 8 und darunter enthalten. Derart geeignete Emulgatoren sind beispielsweise Verbindungen der allgemeinen Formel R¹ - O - R², in der R¹ eine primäre lineare Alkyl-, Alkenyl- oder Acylgruppe mit 20 - 30 C-Atomen und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2 - 5 Hydroxylgruppen ist. Weitere bevorzugt geeignete Emulgatoren mit einem HLB-Wert von 8 und darunter sind die Anlagerungsprodukte von 1 oder 2 Mol Ethylenoxid oder Propylenoxid an Behenylalkohol, Erucylalkohol, Arachidylalkohol oder auch an Behensäure oder Erucasäure. Bevorzugt eignen sich auch die Monoester von C₁₆-C₃₀-Fettsäuren mit Polyolen wie z. B. Pentaerythrit, Trimethylolpropan, Diglycerin, Sorbit, Glucose oder Methylglucose. Beispiele für solche Produkte sind z. B. Sorbitanmonobehenat oder Pentaerythrit-monoerucat.

Weitere geeignete Zusatzstoffe sind Fettstoffe, insbesondere pflanzliche Öle, wie Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls, flüssige Paraffinöle, Isoparaffinöle und synthetische Kohlenwasserstoffe, Di-n-alkylether mit insgesamt 12 bis 36 C-Atomen, z. B. Di-n-octylether und n-Hexyl-n-octylether, Fettsäuren, besonders lineare und/oder verzweigte, gesättigte und/oder ungesättigte C₈₋₃₀-Fettsäuren, Fettalkohole, besonders gesättigte, ein- oder mehrfach ungesättigte, verzweigte oder unverzweigte Fettalkohole mit 6 - 30 Kohlenstoffatomen, Esteröle, das heißt Ester von C₆₋₃₀-Fettsäuren mit C₂₋₃₀-Fettalkoholen, Hydroxycarbonsäurealkylester, Dicarbonsäureester wie Di-n-butyladipat sowie Diolester wie Ethylenglykoldioleat oder Propylenglykoldi(2-ethylhexanoat), symmetrische, unsymmetrische oder cyclische Ester der Kohlensäure mit Fettalkoholen, z. B. Glycerincarbonat oder Dicaprylylcarbonat (Cetiol^{®} CC), Mono,-Di- und Trifettsäureester von gesättigten und/oder ungesättigten linearen und/oder verzweigten Fettsäuren mit Glycerin Wachse, insbesondere Insektenwachse, Pflanzenwachse, Fruchtwachse, Ozokerit, Mikrowachse, Ceresin, Paraffinwachse, Triglyceride gesättigter und gegebenenfalls hydroxylierter C₁₆₋₃₀-Fettsäuren, z. B. gehärtete Triglyceridfette, Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxane, Polyalkylarylsiloxane, ethoxylierte Polydialkylsiloxane sowie Polydialkylsiloxane, die Amin- und/oder Hydroxy-Gruppen enthalten.

Die bevorzugte Einsatzmenge der Fettstoffe beträgt 0,1 - 50 Gew.%, bevorzugt 0,1 - 20 Gew.% und besonders bevorzugt 0,1 - 15 Gew.%, jeweils bezogen auf die gesamte Zusammensetzung.

Weitere geeignete Zusatzstoffe sind Verdickungsmittel, z. B. natürliche und synthetische Tone und Schichtsilikate wie Bentonit, Hectorit, Montmorillonit oder Laponite^{®}, oder anionische Polymere aus Acrylsäure, Methacrylsäure, Crotonsäure, Maleinsäureanhydrid und 2-Acrylamido-2-methylpropansulfonsäure, wobei die sauren Gruppen ganz oder teilweise als Natrium-, Kalium-, Ammonium-, Mono- oder Triethanolammonium-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel^{®}305, Simulgel^{®}600, Simulgel^{®} NS und Simulgel^{®} EG der Firma SEPPIC. Weitere besonders bevorzugte anionische Homo- und Copolymere sind unvernetzte und vernetzte Polyacrylsäuren. Solche Verbindungen sind zum Beispiel die Handelsprodukte Carbopol^{®}. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀₋₃₀-Carbonsäuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342 und ETD 2020 (ex B.F. Goodrich).

Geeignete nichtionische Polymere sind beispielsweise Polyvinylalkohole, die teilverseift sein können, z. B. die Handelsprodukte Mowiol^{®} sowie Vinylpyrrolidon/Vinylester-Copolymere und Polyvinylpyrrolidone, die z. B. unter dem Warenzeichen Luviskol^{®} (BASF) vertrieben werden. Weitere geeignete Zusatzstoffe sind Antioxidantien, Konservierungsmittel, Lösungsmittel wie Ethanol, Isopropanol, Ethylenglykol, Propylenglykol, Propylenglykolmonoethylether, Glycerin und Diethylenglykol, Adsorbentien und Füllstoffe, wie Talkum und Veegum^{®}, Parfümöle, Pigmente sowie Farbstoffe zum Anfärben des Mittels, Substanzen zur Einstellung des pH-Wertes, Komplexbildner wie EDTA, NTA, β-Alanindiessigsäure und Phosphonsäuren, Trübungsmittel und Perlglanzmittel wie Ethylenglykolmono- und -distearat und Treibmittel wie Propan-Butan-Gemische, N₂O, Dimethylether, CO₂ und Luft.

Die nachfolgenden Beispiele sollen den Gegenstand der Erfindung erläutern, ohne ihn hierauf zu beschränken.

**1. Öl-in-Wasser-Emulsionen**

| | 1 | 2 |
|---|---|---|
| Montanov 68 | 5,00 | 5,00 |
| Myritol 318 | 5,00 | 5,00 |
| Shea Butter | 0,50 | 0,50 |
| Novata AB | 2,00 | 2,00 |
| Lanette O | 1,00 | 1,00 |
| Cutina MD | 2,00 | 2,00 |
| Baysilone-Öl M 350 | 0,50 | 0,50 |
| Tocopheryl acetate | 0,50 | 0,50 |
| Hydrogenated Palm Glycerides Citrate | 0,25 | 0,25 |
| Parsol SLX | - | - |
| Neo Heliopan Hydro | 2,00 | 2,00 |
| Octocrylene | - | - |
| Octyl Triazone | 5,00 | - |
| Octylsalicylate | - | - |
| Diethylamino Hydroxybenzoyl Hexyl Benzoate | - | - |
| Buthyl-Methoxydibenzoylmethane | 1,80 | 1,80 |
| TiO2 | - | 2,00 |
| Propylparaben | 0,20 | 0,20 |
| Sodium Carbomer | 0,50 | 0,50 |
| Hexandiol | 6,00 | 6,00 |
| Talc | 0,50 | 0,50 |
| Methylparaben | 0,20 | 0,20 |
| Glycerol | 4,50 | 4,50 |
| L-Carnitine | - | - |
| Betain | 0,50 | 0,50 |
| Phytokine | 5,00 | 5,00 |
| Trisodium NTA | 0,10 | 0,10 |
| Phenoxyethanol | 0,40 | 0,40 |
| Hydrovance | 2,50 | 8,60 |
| Sepivinol R | 1,00 | 0,30 |
| Coenzym Q 10 | 0,10 | 0,10 |
| Fucogel 1000 | 2,00 | - |
| Val-Trp | 0,01 | - |
| Perfume | 0,40 | 0,40 |
| Aqua | ad 100 | ad 100 |

**Tagescremes**

| | 7 | 8 |
|---|---|---|
| Cetearyl Alkohol /Cetearyl Glucoside | 5,00 | 5,00 |
| Caprylic/Capric Triglyceride | 5,00 | 5,00 |
| Shea Butter | 0,50 | 0,50 |
| Cocoglycerides | 2,00 | 2,00 |
| Cetearyl Alkohol | 1,00 | 1,00 |
| Glyceryl Stearate | 2,00 | 2,00 |
| Dimethicone | 0,50 | 0,50 |
| Tocoperyl acetate | 0,50 | 0,50 |
| Hydrogenated Palm Glycerides Citrate | 0,25 | 0,25 |
| Polysilicone-15 | | |
| Phenylbenzimidazole Sulfonic Acid | 2,00 | 2,00 |
| Octocrylene | | |
| Octyl Triazone | 5,00 | |
| Octylsalicylate | | |
| Diethylamino Hydroxybenzoyl Hexyl Benzoat | | |
| Buthyl-Methoxydibenzoylmethane | 1,80 | 1,80 |
| TiO2 | | 2,00 |
| Propylparaben | 0,20 | 0,20 |
| Sodium Carbomer | 0,50 | 0,50 |
| Hexandiole | 6,00 | 6,00 |
| Talc | 0,50 | 0,50 |
| Methylparaben | 0,20 | 0,20 |
| Glycerol | 4,50 | 4,50 |
| L-Carnitine | - | - |
| Betain | 0,50 | 0,50 |
| Hydrolyzed Soy Protein | 5,00 | 5,00 |
| Trisodium NTA | 0,10 | 0,10 |
| Phenoxyethanol | 0,40 | 0,40 |
| (2-Hydroxyethyl)urea | 2,50 | 8,60 |
| Sepivinol R | 1,00 | 0,30 |
| Coenzym Q 10 | 0,10 | 0,10 |
| Fucogel 1000 | 2,00 | - |
| Val-Trp | 0,01 | - |
| Perfume | 0,40 | 0,40 |
| Aqua | ad.100 | ad. 100 |

**Liste der verwendeten Handelsprodukte**

| Handelsname | INCI-Bezeichnung | Lieferant/Hersteller |
|---|---|---|
| | | |
| Baysilone-Öl M 350 | Dimethicone | Bayer |
| Calmosensine | Acetyl Dipeptide-1 Cetylester | Sederma |
| Caomint | Propylene Glycol, Aqua, Mentha piperita, Theobroma cacao | Solabia |
| Cutina MD | Glyceryl Stearate | Cognis |
| Dow Corning 200 Fluid (0,65 cSt) | Dimethicone | Dow Corning |
| Dow Corning 9040 | Cyclomethicone/ Dimethicone Crosspolymer | Dow Corning |
| Dry Flo Plus | Aluminium Starch Octenylsuccinate | National Starch |
| DSH CN | Water, Dimethylsilanol Hyaluronate | Exsymol |
| Ederline H | PEG-40 Hydrogenated Castor Oil, PPG-2-Ceteareth-9, Pyrus Malus (Apple) Fruit Extract | Seporga |
| LANETTE^{®} O | Cetearyl Alcohol | Cognis |
| Montanov 68 | Cetearyl Alkohol, Cetearyl Glucoside | Seppic |
| Montanov L | C14-22 Alcohols/C12-20 Alkyl Glucoside | Seppic |
| Myritol 318 | Caprylic/Capric Triglyceride | Cognis |
| Neo Heliopan Hydro | Phenylbenzimidazole sulfonic acid | Symrise |
| Novata AB | Cocoglycerides | Cognis |
| Omega-CH-Aktivator | Tripeptide-1 | GfN |
| Parsol SLX | Polysilicone-15 | DSM |
| Phytokine | HYDROLYZED SOY PROTEIN | Coletica |
| Sepivinol R | Polyphenolreicher Extrakt aus Rotwein | Seppic |
| Sveltine LHYA923S | AQUA (WATER), BUTYLENE GLYCOL, CARNITINE, LECITHIN, CAFFEINE, CARBOMER, ESCULIN, CENTELLA ASIATICA EXTRACT, ATELOCOLLA-GEN, SODIUM CHONDROITIN SULFATE | Coletica |
| Vegetal Filling Spheres | CAPRYLIC/CAPRIC TRIGLYCERIDE, SILICA DIMETHYL SILYLATE, HYDROLYZED WHEAT PROTEIN, BUTYLENE GLYCOL, PHENOXYETHANOL,METHYLPARABEN,ETHYLPARA-BEN, PROPYLPARABEN, BUTYLPARA-BEN, ISOBUTYLPARABEN | Coletica |

## Patentansprüche

1. Kosmetische oder dermatologische topische Zusammensetzungen, enthaltend in einem geeigneten kosmetischen oder dermatologischen Träger mindestens einen hydroxyalkylsubstituierten Harnstoff der allgemeinen Formel (A) 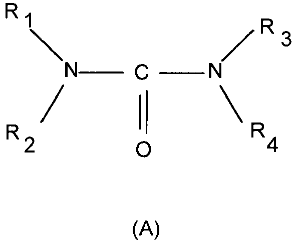
in der R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ - R₄ eine C₂-C₆-Hydroxyalkyl-Gruppe, die mit 1 bis 5 Hydroxylgruppen oder C₁-C₄-Hydroxyalkyl-Gruppen substituiert ist, darstellt,
sowie mindestens einen Wirkstoff, ausgewählt aus natürlichen Betainverbindungen, wobei die natürliche Betainverbindung ausgewählt ist aus Betain (Me₃N⁺-CH₂-COO⁻) mit Me = Methyl.

2. Zusammensetzung gemäß Anspruch 1, **dadurch gekennzeichnet, dass** die Verbindung gemäß Formel (A) ausgewählt ist aus (2-Hydroxyethyl)harnstoff.

3. Zusammensetzung gemäß Anspruch 1 oder 2, **dadurch gekennzeichnet, dass** mindestens ein alkyl- oder hydroxyalkylsubstituierter Harnstoff gemäß Formel (A), insbesondere (2-Hydroxyethyl)harnstoff, in einer Gesamtmenge von 0,01 - 50 Gew.-%, bevorzugt 0,1 - 20 Gew.-%, besonders bevorzugt 1 -10 Gew.-% und außerordentlich bevorzugt 2 - 5 Gew.-%, bezogen auf die gesamte Zusammensetzung, enthalten ist.

4. Zusammensetzung gemäß Anspruch 1 - 3, **dadurch gekennzeichnet, dass** die mindestens eine natürliche Betainverbindung in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten ist.

5. Verwendung einer Zusammensetzung gemäß einem der Ansprüche 1 - 4 zur nicht-therapeutischen, kosmetischen Behandlung und/oder Minimierung von
- Hautfalten und -fältchen,
- den Anzeichen der intrinsischen und extrinsischen Hautalterung,
- trockener Haut.

6. Nicht-therapeutisches Verfahren zur nicht-therapeutischen, kosmetischen Behandlung und/oder Minimierung von
- Hautfalten und -fältchen,
- trockener Haut,
**dadurch gekennzeichnet, dass** eine kosmetische oder dermatologische topische Zusammensetzung gemäß einem der Ansprüche 1 - 4 auf die Haut aufgetragen wird.

## Claims

1. Cosmetic or dermatological topical compositions containing, in a suitable cosmetic or dermatological carrier, at least one hydroxyalkyl-substituted urea of the general formula (A) 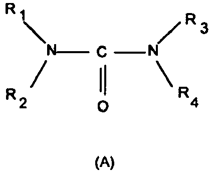
in which, independently of one another, R₁, R₂, R₃ and R₄ represent a hydrogen atom, a methyl, ethyl, n-propyl, isopropyl, n-butyl, secondary butyl, tertiary butyl or C₂-C₆ hydroxyalkyl group which is substituted with 1 to 5 hydroxyl groups or C₁-C₄ hydroxyalkyl groups, with the condition that at least one of the functional groups R₁-R₄ represents a C₂-C₆ hydroxyalkyl group which is substituted with 1 to 5 hydroxyl groups or C₁-C₄ hydroxyalkyl groups,
and at least one active ingredient, selected from natural betaine compounds, wherein the natural betaine compound is selected from betaine (Me₃N⁺-CH₂-COO⁻), where Me = methyl.

2. The composition according to claim 1, **characterized in that** the compound according to formula (A) is selected from (2-hydroxyethyl)urea.

3. The composition according to claim 1 or 2, **characterized in that** it contains at least one alkylsubstituted or hydroxyalkyl-substituted urea according to formula (A), in particular (2-hydroxyethyl)urea, in a total amount of from 0.01-50 wt.%, preferably from 0.1-20 wt.%, more preferably from 1-10 wt.%, and most preferably from 2-5 wt.%, based on the total composition.

4. The composition according to claims 1-3, **characterized in that** it contains the at least one natural betaine compound in a total amount of from 0.05 to 5 wt.%, preferably from 0.1 to 3 wt.%, most preferably from 0.5 to 2 wt.%, in each case based on the total composition.

5. A use of a composition according to one of claims 1-4 for the non-therapeutic, cosmetic treatment and/or minimization of
- folds and wrinkles in the skin,
- the signs of intrinsic and extrinsic skin aging,
- dry skin.

6. A non-therapeutic method for the non-therapeutic, cosmetic treatment and/or minimization of
- folds and wrinkles in the skin,
- dry skin,
**characterized in that** a cosmetic or dermatological topical composition according to one of claims 1-4 is applied to the skin.

## Revendications

1. Compositions cosmétiques ou dermatologiques topiques contenant, dans un vecteur cosmétique ou dermatologique adéquat, au moins une urée hydroxyalkyl-substituée de formule générale (A) : 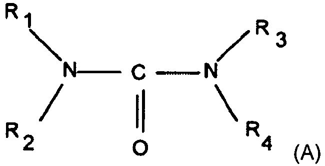
où R₁, R₂, R₃ et R₄ représentent, indépendamment les uns des autres, un atome d'hydrogène, un groupe méthyle, éthyle, n-propyle, isopropyle, n-butyle, s-butyle, t-butyle ou C₂₋₆-hydroxyalkyle, substitué par 1 à 5 groupes hydroxy ou C₁₋₄-hydroxyalkyles, à condition qu'au moins un des résidus R₁ à R₄ représente un groupe C₂₋₆-hydroxyalkyle substitué par 1 à 5 groupes hydroxy ou C₁₋₄-hydroxyalkyles,
ainsi qu'au moins une substance active choisie parmi des composés naturels de la bétaïne, le composé naturel de la bétaïne étant choisi parmi la bétaïne (Me₃N⁺-CH₂-COO⁻) avec Me = méthyle.

2. Composé selon la revendication 1, **caractérisé en ce que** le composé de formule (A) est choisi comme la (2-hydroxyéthyl)urée.

3. Composé selon la revendication 1 ou 2, **caractérisé en ce qu'**elle contient au moins une urée alkylsubstituée ou hydroxyalkylsubstituée de formule (A), en particulier la (2-hydroxyéthyl)urée, à hauteur de 0,01 à 50 % en poids, préférentiellement de 0,1 à 20 % en poids, particulièrement préférentiellement de 1 à 10 % en poids et tout particulièrement préférentiellement de 2 à 5 % en poids, rapporté à la composition totale.

4. Composé selon une des revendications 1 à 3, **caractérisé en ce qu'**elle contient au moins un composé naturel de la bétaïne à hauteur de 0,05 à 5 % en poids, préférentiellement de 0,1 à 3 % en poids, particulièrement préférentiellement de 0,5 à 2 % en poids, rapporté à la composition totale.

5. Composé selon une des revendications 1 à 4 pour le traitement cosmétique non-thérapeutique et/ou la minimisation :
- des rides et ridules,
- des signes du vieillissement intrinsèque et extrinsèque de la peau,
- de la sécheresse cutanée.

6. Procédé non-thérapeutique pour le traitement cosmétique non-thérapeutique et/ou la minimisation :
- des rides et ridules,
- de la sécheresse cutanée,
**caractérisé par** l'application sur la peau d'une composition cosmétique ou dermatologique topique selon une des revendications 1 à 4.
